# EUROPEAN PATENT SPECIFICATION

(11) **EP 1 662 908 B1**
(45) Date of publication and mention of the grant of the patent: **15.07.2009**
(21) Application number: 04740254.0
(22) Date of filing: 24.06.2004
(51) Int. Cl.: A23L 1/302, A23L 1/304, A23L 1/305, A61K 31/00

(54) **COMPOSITION AND METHOD FOR FACILITATING BONE HEALING**
ZUSAMMENSETZUNG UND VERFAHREN ZUR FÖRDERUNG DER KNOCHENHEILUNG
COMPOSITION ET PROCEDE FACILITANT LA GUERISON OSSEUSE

(30) Priority: 05.09.2003 US 657019
(43) Date of publication of application: 07.06.2006
(73) Proprietor: Rath, Matthias, Dr. med., 6422 PC Heerlen (NL)
(72) Inventor: RATH, Matthias, 6422 PC Heerlen (NL); NETKE, Shrirang, Shivaji Nagar, Nagpur, MS 440010 (IN); ROOMI, Waheed, M., Sunnyvale, CA 94086 (US); IVANOV, Vadim, Castro Valley, CA 94552 (US); NIEDZWIECKI, Aleksandra, San Jose, CA 95129 (US)
(74) Representative: Sacht-Gorny, Gudrun
(86) International application number: PCT/EP2004/006841
(87) International publication number: WO 2005/023020

(56) References cited:
- DE-U- 20 207 569
- US-A- 5 232 709
- DATABASE WPI Section Ch, Week 198725 Derwent Publications Ltd., London, GB; Class B05, AN 1987-176219 XP002293029 & SU 1 268 169 A (AKTYUBINSK MED INST) 7 November 1986 (1986-11-07)
- DATABASE WPI Section Ch, Week 200377 Derwent Publications Ltd., London, GB; Class B06, AN 2003-814195 XP002293030 & CN 1 430 970 A (MA S) 23 July 2003 (2003-07-23)

## Description

The present invention generally relates to nutritional compositions for facilitating bone healing and uses thereof.

Bone is a dynamic living tissue and is continuously being replenished by resorption and deposition of bone matrix. The stability of bone depends upon the underlying connective tissue. Oxlund H. *et al.* has shown that optimally structured collagen is more important for bone strength than bone compactness and its calcium saturation (Bone 1996; 19:479-84). A concentration of cross-links between collagen strands appeared 30% less in bone affected by osteoporosis. Knot L*. et al.* has shown that collagen structure and spatial organization of its fiber network is critical for deposition of minerals and compactness in the bone, and that the micro-architecture of collagen determines bone strength (Bone 1998; 22:181-7). Savvas M*. et al*. has shown that a loss of collagen caused by malnutrition is a major factor in loss of bone mass (J. Obstetr. Gynecol. 1989; 96:1392-4). The anorexic women in the study show a lowered bone density of 18% in the spine and 25% in the femur. The decrease in bone mass is associated with a 22% decrease in skin collagen.

The healing process after bone fracture is an orderly process that involves multiple phases including: i) hematoma formation; ii) fibro-cartilaginous callus formation; iii) bony callus formation; and iv) bone remodeling. During the healing process, pluripotential cells in the vicinity of the bone fracture differentiate into osteoblasts and chondrocytes. Osteoblasts origin form osteoid tissues and they lay down collagen fibers. Chondrocytes give rise to hypertrophic chondrocytes that deposit a mineralized matrix to form calcified cartilage, which is then remodeled into compact bone.

Despite advances in orthopaedic techniques, healing of bone fractures is a lengthy process, often requires weeks if not months. A patient often suffers a severe restriction of movement for several weeks. Facilitating bone healing and fracture repair (i.e., reducing healing time) would be a great relief to the patient. This is particularly desirable in adolescents because this age group of individuals has the lowest compliance with doctor recommendations. When doctors recommend that an adolescent use crutches for a certain period of time, they often remove themselves from crutches much earlier than recommended.
U.S. Pat. 6,258,778 discloses a method of enhancing bone and cartilage repair by administering angiotensin and its analogues. U.S. Pat. 5,502,074 discloses a method of facilitating bone healing using benzothiophenes. The safety of use of these drugs are not established. For example, angiotensin is known to exert potent cardiovascular and renal effects, and its use in patients with heart or renal failure may be limited.
U.S. Pat. 6,061,597 discloses the application of resonant frequency stimulation to promote fracture healing. U.S. Pat. 6,290,714 discloses a low level laser therapy in treating bone fracture. The effectiveness of these approach is not shown and requires expensive medical office visits and/or computer equipment. None of these methods has been clinically proven.
U.S. Pat. 5,232,709 discloses a nutritional supplement having a large dose of calcium in treating bone loss. Administering to a bone fractured individual with a large dose of calcium would cause mineralization of the bone tissue, rather than supplementing bone collagen. The increased bone mineralization causes further hardening of bone. The affected bone becomes more brittle over time, making it prone to compound fractures and shattering under stress.

There is a long felt need to provide a safe, convenient, affordable and effective approach to facilitate bone healing (i.e., reduce healing time of bone fractures) in humans.

Thus, the technical problem underlying the present invention must be seen as the provision of means and methods to comply with this need. This technical problem is solved by the embodiments characterized in the claims.

Accordingly, the present invention relates to a nutritional composition comprising lysine, proline, ascorbic acid, copper, and vitamin B₆. The nutritional composition is suitable for human use and is effective in facilitating bone healing. The nutritional composition is also suitable for animal use. Preferably, said animal is mammal, most preferably a dog, cat or horse.

Preferably, the nutritional composition contains 27-34 % wt lysine, 14-15 % wt proline, and 42-47 % wt ascorbic acid.

Preferably, the nutritional composition is administered orally. Preferably, the recommended amount is 1,010 mg - 8 gram lysine, 560 mg - 4 gram proline, 1,500 mg - 9 gram ascorbic acid, 2 µg - 6 mg copper, and 0.5 mg - 10 mg vitamin B₆. More preferably, a recommended amount is 230 mg - 10 gram lysine, 120 mg - 5 gram proline, 360 mg - 15 gram ascorbic acid, 1.5 µg - 20 mg copper, and 0.2 mg - 20 mg vitamin B₆. Most preferably, a recommended amount is 1,010 mg lysine, 560 mg proline, 1,500 mg ascorbic acid, 330 µg copper and 10 mg vitamin B₆.

Preferably, the nutritional composition is a daily dosage (based on a human subject of average body weight of 72 kg) of 3.2 - 139 mg/kg lysine, 1.7 - 69.4 mg/kg proline, 5 - 208.3 mg/kg ascorbic acid, 0.02 - 278 µg/kg copper, 2.78 - 279 µg/kg vitamin B₆.

More preferably, the nutritional composition is a daily dosage of 14 - 111 mg/kg lysine, 7.8 - 55.6 mg/kg proline, 20.8 - 125 mg/kg ascorbic acid, 0.03 - 83.3 µg/kg copper, and 6.94 -139 . µg/kg vitamin B₆.

Most preferably, the nutritional composition is a daily dosage of 14 mg/kg lysine, 7.8 mg/kg proline, 20.8 mg/kg ascorbic acid, 4.6 µg/kg copper, 139 µg/kg vitamin B₆.

Preferably, the nutritional composition further comprises vitamin A, vitamin D₃, vitamin E, vitamin B₁, vitamin B₂, niacin, folic acid, vitamin B₁₂, biotin, pantothenic acid, calcium, phosphorus, magnesium, zinc, selenium, manganese, chromium, molybdenum, potassium, citrus fruit peel bioflavanoids, arginine, cysteine, inositol, carnitine, coenzyme Q₁₀, and pycnogenol.

Preferably, the recommended amount is 67 µg -100 mg vitamin A, 0.7 µg - 50 µg vitamin D₃, 0.7 µg - 50 µg vitamin E, 1.4 mg - 8 mg vitamin B₁, 1.4 mg - 8 mg vitamin B₂, 9 mg - 250 mg niacin, 18 µg - 500 µg folic acid, 4 µg - 100 µg vitamin B₁₂, 13 µg - 400 µg biotin, 8 mg - 100 mg pantothenic acid, 7 mg - 40 mg calcium, 3 mg - 300 mg phosphorus, 40 mg - 200 mg magnesium, 0.5 mg - 10 mg zinc, 20 µg - 300 µg selenium, 0.8 mg - 15 mg manganese, 2 µg - 200 µg chromium, 0.8 µg - 100 µg molybdenum, 4 mg - 300 mg potassium, 20 mg - 500 mg citrus fruit peel bioflavanoids, 10 mg - 500 mg arginine, 10 mg - 400 mg cysteine, 5 mg - 400 mg inositol, 5 mg - 400 mg carnitine, 1.6 mg - 70 mg coenzyme Q₁₀, and 1.6 mg - 70 mg pycnogenol.

More preferably, the recommended amount is 166 µg -50 mg vitamin A, 1.65 µg -20 µg vitamin D₃, 1.65 µg - 20 µg vitamin E, 3.5 mg - 7 mg vitamin B₁, 3.5 mg - 7 mg vitamin B₂, 22.5 mg - 100 mg niacin, 45 µg - 300 µg folic acid, 10 µg - 50 µg vitamin B₁₂, 32 µg - 300 µg biotin, 20 mg - 60 mg pantothenic acid, 17 mg - 35 mg calcium, 7 mg - 100 mg phosphorus, 50 mg - 100 mg magnesium, 3 mg - 8 mg zinc, 30 µg - 250 µg selenium, 1 mg - 3.25 mg manganese, 2 µg - 75 µg chromium, 2 µg - 75 µg molybdenum, 8 mg - 200 mg potassium, 50 mg - 250 mg citrus fruit peel bioflavanoids, 100 mg - 300 mg arginine, 80 mg - 200 mg cysteine, 80 mg - 200 mg inositol, 80 mg - 200 mg carnitine, 3 mg - 35 mg coenzyme Q₁₀, and 3 mg-35 mg pycnogenol.

Most preferably, the recommended amount is 333 µg vitamin A, 3.3 µg vitamin D₃, 3.3 µg vitamin E, 7 mg vitamin B₁, 7 mg vitamin B₂, 45 mg niacin, 90 µg folic acid, 20 µg vitamin B₁₂, 65 µg biotin, 40 mg pantothenic acid, 35 mg calcium, 15 mg phosphorus, 40 mg magnesium, 7 mg zinc, 20 µg selenium, 1.3 mg manganese, 10 µg chromium, 4 µg molybdenum, 20 mg potassium, 100 mg citrus fruit peel bioflavanoids, 40 mg arginine, 35 mg cysteine, 35 mg inositol, 35 mg carnitine, 7 mg coenzyme Q₁₀, and 7 mg pycnogenol.

Preferably, the nutritional composition comprises a daily dosage (based on a human subject of average body weight of 72 kg) of 0.9-1,390 µg/kg vitamin A, 0.01-0.694 µg/kg vitamin D₃, 0.01-0.694 µg/kg vitamin E, 19.4-111 µg/kg vitamin B₁, 19.4-11 µg/kg vitamin B₂,125-3,472 µg/kg niacin, 0.25-6.94 µg/kg folic acid, 0.05-1.39 µg/kg vitamin B₁₂, 0.181-5.56 µg/kg biotin, 111-1,390 µg/kg pantothenic acid, 97.2-555 µg/kg calcium, 42-4,167 µg/kg phosphorus, 555-2,778 µg/kg magnesium, 6.9-139 µg/kg zinc, 0.28-4.17 µg/kg selenium, 111--208.3 µg/kg manganese, 0.03-2.78 µg/kg chromium, 0.01-1.39 µg/kg molybdenum, 55.6-4,167 µg/kg potassium, 278-6.944 µg/kg citrus fruit peel bioflavanoids, 139-6,944 µg/kg. arginine, 135-5,555 µg/kg cysteine, 69-5,555 µg/kg inositol, 69-5,555 µg/kg carnitine, 22.2-972 µg/kg coenzyme Q₁₀, and 22.2-972 µg/kg pycnogenol.

More preferably, the nutritional composition comprises a daily dosage (based on a human subject of average body weight of 72 kg) of 2.31-694 µg/kg vitamin A, 0.023-0.278 µg/kg vitamin D₃, 0.023-0.278 µg/kg vitamin E, 48.6-97.2 µg/kg vitamin B₁, 48.6-97.2 µg/kg vitamin B₂, 312.5-3,190 µg/kg niacin, 0.6-4.17 µg/kg folic acid, 0.14-0.69 µg/kg vitamin B₁₂, 0.444-4.17 µg/kg biotin, 278-833 µg/kg pantothenic acid, 236-903 µg/kg calcium, 97.2-1,390 µg/kg phosphorus, 694-1,390 µg/kg magnesium, 41.7-111 µg/kg zinc, 0.42-3.47 µg/kg selenium, 13.9-45.1 µg/kg manganese, 0.07-2.78 µg/kg chromium, 0.03-1.04 µg/kg molybdenum, 111.1-2,778 µg/kg potassium, 694-3,472 µg/kg citrus fruit peel bioflavanoids, 1,389.4,167 µg/kg arginine, 1,111-2,778 µg/kg cysteine, 1,111-2,778 µg/kg inositol, 1,111-2,778 µg/kg carnitine, 41.7-486 µg/kg coenzyme Q₁₀, and 41.7-486 µg/kg-pycnosenol.

Most preferably, the nutritional composition comprises a daily dosage (based on a human subject of average body weight of 72 kg) of 4.6 µg/kg vitamin A, 0.046 µg/kg vitamin D₃, 0.0469 µg/kg vitamin E, 97.2 µg/kg vitamin. B_{1,} 97.2 µg/kg vitamin B_{2,} 625 µg/kg niacin, 1.25 µpg/kg folic acid, 0.27 µg/kg vitamin B₁₂, 0.9 µg/kg biotin**,** 555 µg/kg pantothenic acid, 486 µg/kg calcium; 208 µg/kg phosphorus, 555 µg/kg magnesium, 97.2 µg/kg zinc, 0.78 µg/kg selenium, 18.1. µg/kg manganese, 0.14 µg/kg chromium, 0.06 µg/kg molybdenum, 277.8 µg/kg potassium, 1,389 µg/kg citrus fruit peel bioflavanoids, 555 µg/kg arginine, 486 µg/kg cysteine, 486 µg/kg inositol, 486 µg/kg carnitine, 97.2 µg/kg enzyme Q₁₀, and 97.2 µg/kg pycnogenol; preferably 0.01-0.694 µg/kg vitamin D₃, 18.1µg/kg manganese, 555 µg/kg arginine, 486 µg/kg cysteine, 4.6 µg/kg vitamin A, 0.046 µg/kg vitamin E, 97.2 µg/kg vitamin B₁, 97.2 µg/kg vitamin B₂, 0.27 µg/kg vitamin B₁₂,625 µg/kg niacin, 1.25 µg/kg folic acid, 0.9 µg/kg biotin, 555 µg/kg pantothenic acid, 486 µ/kg calcium, 208 µg/kg phosphorus, 555 µg/kg magnesium, 97.2 µg/kgzinc, 0.78 µg/kg, selenium, 0.14 µg/kg chromium, 0.06 µg/kg molybdenum, 277.8 µg/kg potassium, 1,389 µg/kg citrus fruit peel bioflavanoids, 486 µg/kg inositol, 486 µg/kg carnitine, 97.2 µg/kg coenzyme Q₁₀, and 97.2 µg/kg pycnogenol.

The present invention provides a method for facilitating bone healing in a mammal, comprising the step of administering to a mammal in need thereof an effective amount of a nutritional composition comprising lysine, proline, ascorbic acid, copper, and vitamin B₆,

The present invention further provides a method for facilitating bone healing in a mammal comprises the step of administering to a mammal in need thereof an effective amount of a nutritional composition further comprising vitamin A, vitamin D₃, vitamin E, vitamin B₁, vitamin B₂, niacin, folic acid, vitamin B₁₂, biotin, pantothenic acid, calcium, phosphorus, magnesium, zinc, selenium, manganese, chromium, molybdenum, potassium, citrus fruit peel bioflavanoids, arginine, cysteine, inositol, carnitine, coenzyme Q₁₀, and pycnogenol.

Preferably, the mammal is a human.

Preferably, the nutritional composition is effective in reducing healing time for bone fractures. Preferably, the healing, time is reduced > about 5%. More preferably, the healing time is reduced > about 15%. Most preferably, the healing time is reduced >about 50%.

Preferable, the nutritional composition is effective in humans of all ages. Preferably, the nutritional composition is suitable for facilitating bone healing in adults of 31-40 and 41-50 years of age. The nutritional composition provides a 37 % and 40% reduction in healing time respectively. More preferably, the nutritional composition is effective in humans of 10-20 years of age. (i.e., adolescents), which provides a 49% reduction in healing time.

Preferably, the nutritional composition may be administered orally, intravenously, or parenterally.

As used herein, the term "bone healing" refers to the healing of bone fractures. Bone healing shall also encompass the process of bone repair and shall not be limited to healing or accidental bone fractures. Bone healing also concerns surgical intervention of bones such as bone replacement (e.g., hip and knee joint replacement) and bone implantation (e.g., tooth implantation). When a bone is healed, the normal mobility at the fractured bone site is restore and therm is absence of pain elicited by stressing the fracture or by walking and general restoration of efficient and painless functioning of the affected limb at the fracture site.

The term "healing time" refers to the time elapsed from the time When bone fracture occur until the time whey the bone fracture is healed. With respect to the experiments performed in the studies disclosed herein, the healing time is measured for the time elapsed from the time of reduction of fractured bone until the bone is healed. "Reduction" refers to the process of aligning the tips of a fractured bone (e.g., tibia) at the point of fracture in a position to allow fusing, of the fractured bone tips together. "Adolescent" is a human between about 10 and about 20 years of age. "Effective amount" refers to an amount of the present nutritional composition effective in, reducing the healing time of bone fracture. "Pharmaceutically acceptable" refers to carriers, diluents, and excipients that are compatible with the other ingredients of the formulation, and not deleterious to the recipient thereof "% wt" refers to % of a specific ingredient as a % proportion to the total weight of the nutritional composition, for example, 27 % wt of lysine refers to a nutritional composition in which 27 % of the total weight of the nutritional composition is lysine.

The present nutritional composition is suitable for use in a mammal. Preferably, the mammal is a human. Different age groups of humans may exhibit different speeds of bone healing. For example, human of elder age may be easier to suffer from bone fracture (due to decalcification by osteoporosis) and is likely to have a longer healing time. The present nutritional composition is found to be effective in facilitating bone healing in human in general ; and not particularly limited to a particular age group.

The present invention provides a nutritional composition for facilitating bone healing in human, preferably in adolescent individuals, comprising the step of administering to a human in need of treatment an effective amount of the composition comprising lysine, proline, ascorbic acid, copper, and vitamin B₆. Preferably, the nutritional composition contains z % wt lysine, 42-47 % wt ascorbic acid and 14-15 % wt proline.

The present nutritional composition also contains lysine and proline. Lysine and proline are constituents of collagen and proteins in the bone. Lysine and proline may contribute to osteoblast proliferation of alkaline phosphatase, nitric oxide, insulin like growth factor-I, and collagen type I and may be essential for proper bone formation.

Lysine may include lysine salts such as hydroxylysine and hydroxylysine salts. A daily dose of 3.2 - 139 mg/kg lysine is recommended. Preferably, 14 to 111 mg/kg lysine is used; and more preferably, 14 mg/kg lysine is used. For an average individual weighing 72 kg, the daily recommended dosage of lysine is 230 mg to 10 grams; preferably, 1,010 mg to 8 grams; and more preferably 1,010 mg.

Proline is a non-essential amino acid. However, its synthesis in human body could be limited under certain conditions. It has been reported that the stress of fracture lowers non-essential amino acid levels in plasma of elder humans. In such a case, deficiency of proline, a semi-essential amino acid, if any, would adversely affect the healing of fracture, since this amino acid is present in a large proportion in collagen.

Proline may include proline salts such as hydroxyproline and hydroxyproline salts. A daily dose of 1.7- 69.4 mg/kg proline is recommended. Preferably, 7.8 to 56 mg/kg is used; and more preferably, 7.8 mg/kg is used. For an average individual weighing 72 kg, the daily recommended dosage of proline is 120 mg to 5 grams; preferably, 560 mg to 4 grams; and more preferably 560 mg.

The present nutritional composition contains ascorbic acid. Ascorbic acid may promote the progressive development of osteoblast phenotype and facilitate bone healing and it is also necessary for the differentiation and proliferation of osteogenic and chondrogenic cells.

Ascorbic acid and vitamin C are used interchangeably. The term ascorbic acid encompasses ascorbic acid and salts thereof. Preferably, the ascorbic acid to be applied in accordance with the present invention is calcium ascorbate, magnesium ascorbate or ascorbyl palmitate. A daily dose of 5 - 208 mg/kg ascorbic acid is recommended. Preferably, 20.8 - 125 mg/kg is used; and more preferably, 20.8 mg/kg is used. For an average individual weighing 72 kg, the daily recommended dosage of ascorbic acid is 360 mg to 15 grams; preferably, 1,500 mg to 9 grams; and more preferably 1,500 mg.

The present invention further provides a nutrient composition further comprising minerals and/or trace elements. Trace elements may help to catalyze the production of these macromolecules needed for connective tissue structure and function. Preferred trace elements in accordance with the present invention are iron, iodine, copper, zinc, manganese, cobalt, molybdenum, selenium, chromium, nickel, tin, fluorine or vanadium.

Copper, as a cofactor for lysyl oxidase, is essential for intra- and intermolecular cross-links in collagen. Copper deficit has been shown to impair the mechanical strength of bone. It was hypothesized that a relatively large quantity of ascorbic acid, vitamin B₆, L-lysine and L-proline, together with copper, would have a pronounced effect on bone collagen health and function to produce a marked difference in healing time between fractured bones of a supplement group and a placebo group.

Copper compounds may include copper glycinate. A daily dose of 0.02 to 278 µg/kg copper is recommended. Preferably, 0.03 to 83 µg/kg is used; and more, preferably, 4.6 µg/kg is used. For an average individual weighing 72 kg, the daily recommended dosage is 1.5 µg to 20 mg; preferably 2 µg to 6 mg; and more preferably, 330 µg.

Vitamin B₆ is of importance in bone healing, as it is instrumental in providing reducing equivalents necessary for mineralization. Vitamin B₆ deficiency caused marked diminution in glucose 6-phosphate dehydrogenase activity in perisoteal bone formation and in developing callus. It also caused changes in the bone suggestive of imbalance between osteoblasts and osteoclasts.

Vitamin B₆ compounds may include pryridosine HCl. A daily dose of 2.8 to 279 µg/kg vitamin B₆ is recommended. Preferably, 7 to 139 µg/kg vitamin B₆ is used; and more preferably, 139 µg/kg is used. For an average individual weighing 72 kg, the daily recommended dosage of vitamin B₆ is 0.2 to 20 mg; preferably, 0.5 to 10 mg; and more preferably, 10 mg.

Certain ingredients of the nutritional composition according to the invention are present at a high amount. Specifically, lysine is present between 27-34 % wt (preferably at 28 - 33 % wt); proline is present between 14 - 16 % wt (preferably 15-16 % wt); and ascorbic acid is present between 42-47 % wt (preferably at 43-46 % wt).

Unexpectedly, it has been found in accordance with the present invention that a nutritional composition as specified herein efficiently facilitates bone healing (i.e. it reduces the healing time) of bone fractures in humans, particularly in adolescents. Advantageously, administration of the nutritional composition of the present invention does not merely facilitate bone healing efficiently, but also improves general well being, and is cost effective.

A recommended daily oral dosage includes 3.2-139 mg/kg lysine, 1.37-69 mg/kg proline, 5-208 mg/kg ascorbic acid, 2.78-279 µg/kg vitamin B₆, and 0.02-278 µg/kg copper. Preferably, the recommended daily oral dosage is: 14-111 mg/kg lysine, 7.8 - 56 mg/kg proline, 20.8-125 mg/kg ascorbic acid, 6.9-139 µg/kg vitamin B₆, and 0.03-83 µg/kg copper. More preferably, the recommended daily oral dosage is: 14 mg/kg lysine, 7.8 mg/kg proline, 20.8 mg/kg ascorbic acid, 139 µg/kg vitamin B₆, 4.6 µg/kg copper. Preferably, the nutritional composition is administered 3 tablets per day (i.e., one tablet in morning, one tablet in afternoon and one tablet at night).

Several other dietary components, such as: protein, calcium, magnesium, zinc, copper, iron, fluoride, and vitamins D, A and K, are required for normal bone metabolism. All of these nutrients impact fracture healing, some more directly than others. The trauma of bone fracture was shown to cause a decrease in copper, manganese, and zinc levels in liver, suggesting increased requirement of these minerals after bone fracture.

The present nutritional composition may further comprise vitamin D₃, manganese, arginine, cysteine, vitamins A, E, B₁, B₂, B₁₂, niacin, folic acid, biotin, pantothenic acid, calcium, phosphorus, magnesium, zinc, selenium, chromium, molybdenum, potassium, citrus fruit peel bioflavanoids, inositol, carnitine, coenzyme Q₁₀, and pycnogenol.

The particular dosage of the present nutritional composition required to facilitate bone healing (i.e., reducing healing time) will depend on the severity of the medical condition, the route of administration and the particular subject being treated. The nutritional composition of the present invention may be administered by a variety of routes including oral, intravenous, or parenteral administration. Preferably, the nutritional composition is in unit dosage form, e.g. tablets or capsules. Thus, the present nutritional composition is recommended to be administered as an orally tablet preparation.

A daily recommended dosage (based on a human subject of average body weight of 72 kg) may further contain 0.9-1,390 µg/kg vitamin A, 0.01-0.694 µg/kg vitamin D₃, 0.01-0.694 µg/kg vitamin E, 19.4-111 µg/kg vitamin B₁. 19.4-111 µg/kg vitamin B₁. 125-3,472 µg/kg niacin, 0.25-6.94 µg/kg folic acid, 0.05-1.39 µg/kg vitamin B₁₂, 0.181-5.56 µg/kg biotin, 111-1,390 µg/kg pantothenic acid, 97.2-555 µg/kg calcium, 42-4,167 µg/kg phosphorus, 555-2,778 µg/kg magnesium, 6.9-139 µg/kg zinc, 0.28-4.17 µg/kg selenium, 11.1-208.3 µg/kg manganese, 0.03-2.78 µg/kg chromium, 0.01-1.39 µg/kg molybdenum, 55.6-4,167 µg/kg potassium, 278-6,944 µg/kg citrus fruit peel bioflavanoids, 139-6,944 µg/kg arginine, 135-5,555 µg/kg cysteine, 69-5,555 µg/kg inositol, 69-5,555 µg/kg carnitine, 22.2-972 µg/kg coenzyme Q₁₀, and 22.2-972 µg/kg pycnogenol.

Preferably, the daily recommended dosage (based on a human subject of average body weight of 72 kg) may further contain 2.31-694 µg/kg vitamin A, 0.023-0.278 µg/kg vitamin D₃, 0.023-0.278 µg/kg vitamin E, 48.6-97.2 µg/kg vitamin B_{1,} 48.6-97.2 µg/kg vitamin B_{2,} 312.5-3,190 µg/kg niacin, 0.6-4.17 µg/kg folic acid, 0.14-0.69 µg/kg vitamin B₁₂, 0.444-4.17 µg/kg biotin, 278-833 µg/kg pantothenic acid, 236-903 µg/kg calcium, 97.2-1,390 µg/kg phosphorus, 694-1,390 µg/kg magnesium, 41.7-111 µg/kg zinc, 0.42-3.47 µg/kg selenium, 13.9-45.1 µg/kg manganese, 0.07-2.78 µg/kg chromium, 0.03-1.04 µg/kg molybdenum, 111.1-2,778 µg/kg potassium, 694-3,472 µg/kg citrus fruit peel bioflavanoids, 1,389-4,167 µg/kg arginine, 1,111-2,778 µg/kg cysteine, 1,111-2,778 µg/kg inositol, 1,111-2,778 µg/kg carnitine, 41.7-486 µg/kg coenzyme Q₁₀, and 41.7-486 µg/kg pycnogenol._{.}

Most preferably, the nutritional composition may further include a daily dosage (based on a human subject of average body weight of 72 kg) of 4.6 µg/kg vitamin A, 0.046 µg/kg vitamin D₃, 0.046 µg/kg vitamin E, 97.2 µg/kg vitamin B_{1,} 97.2 µg/kg vitamin B₂, 625 µg/kg niacin, 1.25 µg/kg folic acid, 0.27 µg/kg vitamin B₁₂, 0.9 µg/kg biotin, 555 µg/kg pantothenic acid, 486 µg/kg calcium, 208 µg/kg phosphorus, 555 µg/kg magnesium, 97.2 µg/kg zinc, 0.78 µg/kg selenium, 18.1 µg/kg manganese, 0.14. µg/kg chromium, 0.06 µg/kg molybdenum, 277.8 µg/kg potassium, 1,389 µg/kg citrus fruit peel bioflavanoids, 555 µg/kg arginine, 486 µg/kg cysteine, 486 µg/kg inositol, 486 µg/kg carnitine, 97.2 µg/kg coenzyme Q₁₀, and 97.2 µg/kg pycnogenol; preferably 0.01-0.694 µg/kg vitamin D₃, 18.1 µg/kg manganese, 555 µg/kg arginine, 486 µg/kg cysteine, 4.6 µg/kg vitamin A, 0.046 µg/kg vitamin E, 97.2 µg/kg vitamin B₁, 97.2 µg/kg vitamin B₂, 0.27 µg/kg vitamin B_{12,} 625 µg/kg niacin, 1.25 µg/kg folic acid, 0.9 µg/kg biotin, 555 µg/kg pantothenic acid, 486 µg/kg calcium, 208 µg/kg phosphorus, 555 µg/kg magnesium, 97.2 µg/kg zinc, 0.78 µg/kg selenium, 0.14 µg/kg chromium, 0.06 µg/kg molybdenum, 277.8 µg/kg potassium, 1,389 µg/kg citrus fruit peel bioflavanoids, 486 µg/kg inositol, 486 µg/kg carnitine, 97.2 µg/kg coenzyme Q₁₀, and 97.2 µg/kg pycnogenol.

The present Nutritional composition may include a pharmaceutically acceptable carrier, diluent, or excipient. The nutritional composition of the present invention can be prepared by procedures known in the art. Respective ingredients may be formulated with common excipients, diluents, or carriers, and formed into tablets, capsules, suspensions, powders, and the like. Examples of excipients, diluents, and carriers include: i) fillers and extenders such as starch, sugars, mannitol, and silicic derivatives; ii) binding agents such as carboxymethyl cellulose and other cellulose derivatives, alginates, gelatin, and polyvinyl-pyrrolidone; iii) moisturizing agents such as glycerol; disintegrating agents such as calcium carbonate and sodium bicarbonate; agents for retarding dissolution such as paraffin; iv) resorption accelerators such as quaternary ammonium compounds; v) surface active agents such as acetyl alcohol, and glycerol monostearate; v) adsorptive carriers such as kaolin and bentonite; and vi) lubricants such as talc, calcium and magnesium stearate, and solid polyethyl glycols.

The nutritional compositions may also be formulated as elixirs or solutions for convenient oral administration or as solutions appropriate for parenteral administration, for example, by intramuscular, subcutaneous or intravenous routes. Ideally the formulation is in the form of a pill, tablet, capsule, lozenge, liquid or similar dosage form. The nutritional compositions may well be suited to formulation as sustained release dosage forms and the like.

### Tablets Preparation

The ingredients listed in Table 1 were formulated to form tablets. The tablets contained the key ingredients of lysine (1,010 mg), proline (560 mg), ascorbic acid (1,500 mg), copper (330 µg) and vitamin B₆ (10 mg).

The tablets further contained additional ingredients including vitamin D₃, manganese, arginine, cysteine, vitamins A, E, B₁, B₂, B₁₂, niacin, folic acid, biotin, pantothenic acid, calcium, phosphorus, magnesium, zinc, selenium, chromium, molybdenum, potassium, citrus fruit peel bioflavanoids, inositol, carnitine, coenzyme Q₁₀, and pycnogenol.

**Table 1 - Serving Size - Three Tablets/Day**

| **Key Ingredients** | **Daily Dosage** | **Daily Dosage per Body Weight** |
|---|---|---|
| L-Lysine (from L-Lysine HCl) | 1,010 mg | 14.0 mg/kg |
| | (28 % wt) | |
| L-Proline | 560 mg | 7.8 mg/kg |
| | (16 % wt) | |
| Ascorbic Acid (Ascorbyl Palmitate, | 1,500 mg | 20.8 mg/kg |
| Calcium Ascorbate, Magnesium Ascorbate) | (42.8 % wt) | |
| Copper (Copper Glycinate) | 330 µg | 4.58 µg/kg |
| | (<0.01 % wt) | |
| Vitamin B₆ (Pyridoxine HCl) | 10 mg | 139 µg/kg |
| | (0.28 % wt) | |
| | | |
| **Additional Ingredients** | | |
| Vitamin A (7.5% Betatene (Henkel)) | 333 µg | 4.6 µg/kg |
| Vitamin D₃ (Cholecalciferol) | 3.3 µg | 0.046 µg/kg |
| Vitamin E (Mixed Covitol) | 3.3 µg | 0.046 µg/kg |
| Vitamin B₁ (Thiamine Mononitrate) | 7 mg | 97.2 µg/kg |
| Vitamin B₂(Riboflavin) | 7 mg | 97.2 µg/kg |
| Niacin (Niacinamide) | 45 mg | 625 µg/kg |
| Folic Acid | 90 µg | 1.25 µg/kg |
| Vitamin B₁₂ (Cyanocobalamin) | 20 µg | 0.27 µg/kg |
| Biotin | 65 µg | 0.90 µg/kg |
| Pantothenic Acid (D-Calcium Pantothenate) | 40 mg | 555 µg/kg |
| Calcium (Gycinate, Ascorbate) | 35 mg | 486 µg/kg |
| Phosphorus (Dicalcium Phosphate) | 15 mg | 208 µg/kg |
| Magnesium (Magnesium Glycinate, Magnesium Ascorbate) | 40 mg | 555 µg/kg |
| Zinc (Zinc Glycinate) | 7 mg | 97.2 µg/kg |
| Selenium (L-Selenomethionine) | 20 µg | 0.78 µg/kg |
| Manganese (Amino Acid Chelate) | 1.3 mg | 18.1 µg/kg |
| Chromium (Chromium Glycanate) | 10 µg | 0.14 µg/kg |
| Molybdenum (Molybdenum Glycinate) | 4 µg | 0.06 µg/kg |
| Potassium (Potassium Proteinate) | 20 mg | 277.8 µg/kg |
| Citrus Fruit Peel Bioflavanoids | 100 mg | 1,389 µg/kg |
| L-Arginine (L-Arginine HCl) | 40 mg | 555 µg/kg |
| L-Cysteine (L-Cysteine Monohydrate HCl) | 35 mg | 486 µg/kg |
| Inositol | 35 mg | 486 µg/kg |
| L-Carnitine (L-Camitine Tartrate) | 35 mg | 486 µg/kg |
| CoEnzyme Q₁₀ | 7 mg | 97.2 µg/kg |
| Pycnogenol | 7 mg | 97.2 µg/kg |
| **TOTAL** | **3.5g** | **48.6 mg/kg** |

| | | |
|---|---|---|
| Body Weight refers to a human subject of average body weight of 72 kg | | |

### Clinical Studies

### Patient Selection:

A randomized double-blind placebo-controlled study was performed. The clinical study was conducted according to the recommendations of the Declaration of Helsinki, its amendments and AMG. The inclusion criteria for patient admission were: (i) unilateral displaced closed or grade I open fractures of tibial shaft; and (ii) age above 10 years. The exclusion criteria for patient admission were: (i) patients who had other major injuries, (ii) patients with cardiopulmonary, rheumatological, neurological or metabolic diseases, (iii) patients with previous injuries which influenced their general functions, (iv) patients with fractures within 5 cm distal to tibial tuberosity or within 5 cm proximal to the ankle joint.

Admitted patients were either receive standard care and placebo or standard care with supplementation with as nutritional supplement comprising lysine, proline, ascorbic acid, copper and vitamin B₆. Qualifying patients, on admission to the study, were clinically examined and the radiographs of the affected limbs were taken, fractures reduced under anesthesia and above knee plaster casts applied.

Efforts were made to ensure that age groups and fracture types were equally distributed in the two groups. Patients were entered in the study from February 2001 until December 2002. A total of 113 patients with unilateral displaced closed or grade I open tibial factures were studied. Admitted patients were given informed consent. Participants were advised that data obtained from the studies would be submitted for publication. Out of these, 54 patients were assigned to the supplemented group and 59 patients were assigned to the placebo group. Table 2 summarizes the age distribution of the total 131 patients.

**Table 2 - Distribution of Patients by Age Groups**

| **Age** | **10-20 years** | **21-30 years** | **31-40 years** | **41-50 years** | **>50 years** | **Total** |
|---|---|---|---|---|---|---|
| **Supplemented** | 2 | 7 | 6 | 3 | 3 | 21 |
| **Group** | (9.5%)* | (33.3%) | (28.6%) | (14.3%) | (14.3%) | (100%) |
| **Placebo Group** | 8 | 10 | 10 | 4 | 4 | 36 |
| | (22.2%) | (27.8%) | (27.8)% | (11.1%) | (11.1%) | (100%) |

| | | | | | | |
|---|---|---|---|---|---|---|
| *Values in parenthesis represent percentage of total patients in the specified age group | | | | | | |

### Clinical Protocol:

All fractures were reduced (closed reduction) under anesthesia and above knee plaster casts applied. The fractured limbs were routinely radiographed before and after reduction. The supplemented group of patients were supplied with the nutritional composition in Table 1 and the placebo group of patients with bottles containing placebo tablets. The nutritional composition listed in Table I were studied to evaluate if the proposed desired supplements can ensure adequacy of nutrients impacting fracture healing. The placebo tablets contained maternal of no medical significance, such as cellulose, fructose etc., but were physically indistinguishable from nutrient tablets. All patients were asked to take one tablet thrice daily (morning, afternoon and night).

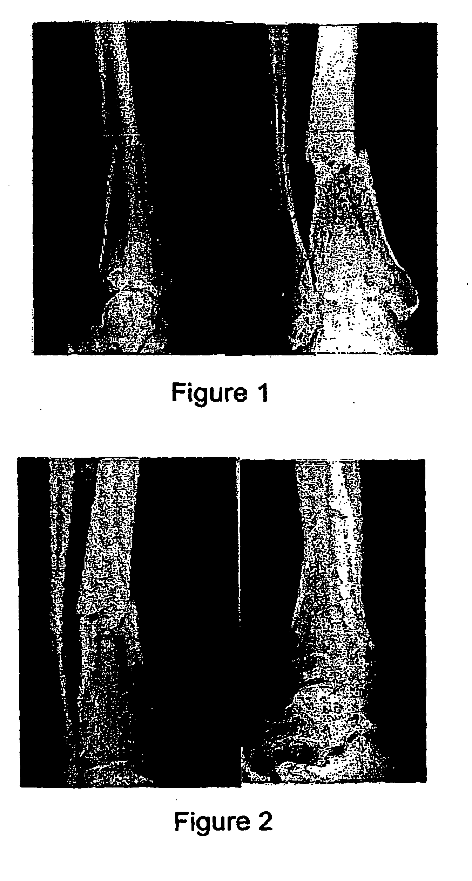

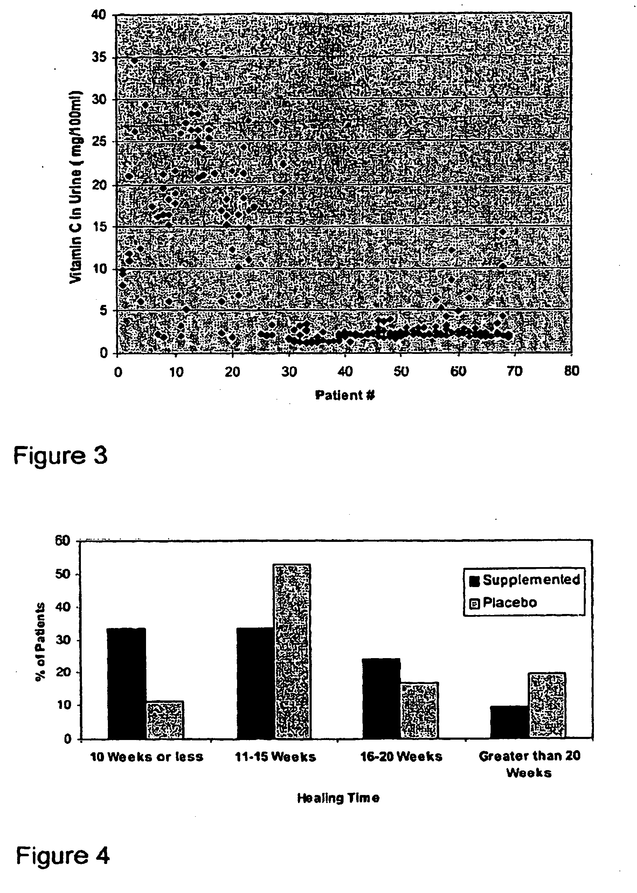

Urine samples of the patients were taken to evaluate their baseline ascorbic acid levels. Blood samples were also taken to assess the baseline calcium levels (Figure 3). The patients were then discharged from the hospital and were asked to return for check-up every four weeks until the treating orthopedic surgeon deemed the fracture healed At each follow-up examination, tibial fractures were radiographed, and patients were clinically examined and rested for urinary vitamin C and blood calcium levels. The radiological examination was done to confirm that the fragments of the fracture remained in reduced position and that callus formation was progressing satisfactorily. The ascorbic acid content of the urine was determined by spectrophotometry and the blood calcium content was determined with commercial kits. Healing was defined as absence of abnormal mobility at the fracture site clinically and absence of pain elicited by stressing the fracture or by walking. Radiographic confirmation of callus formation was used as supporting evidence for healing. (See Figures 1 and 2 for radiographic examples). A healing period of greater than 20 weeks without any surgical intervention was considered delayed healing.

### Statistical Analysis

The results were expressed as means ± standard error for the groups. The Wilcoxon test was performed on the fracture healing times among various groups. Statistical significance was set at P<0.05.

### Results:

29 patients from the supplemented group and 42 patients from the placebo group returned for regular follow-ups until fractures were deemed healed.

Overall, the urinary ascorbic acid content in the supplemented patient group was higher than that in the placebo group (Figure 3). Low urinary ascorbic acid values (below 5 mg/100 ml of urine) were detected in eight supplemented patients and high Ascorbic acid values (above 5 mg/100 ml of urine) in six placebo patients at any one of the check-up visits. These patients were excluded from evaluation. Therefore, only 21 patients in the supplemented group and 36 patients in the placebo group remained for the completion of the study. Blood calcium levels of all participants in the study were within normal limits.

The age distribution of the patients is detailed in Table 2. The age range of the patients in the supplemented group was between 15 to 65 years, with a mean age of 35 years. The age range of the patients in the placebo group was between 12 to 75 years, with a mean age of 32 years.

The healing time of the patients is detailed in Table 4. The mean healing time for the patients in the supplemented group was 14.0±1.1 weeks. The mean healing time for the patients in the placebo group was 16.9± 1.2 weeks. These data show that the overall mean healing time in the supplemented group is about three weeks shorter than that for the placebo group (i.e., 17.2 %). This difference attains statistical significance at t=1.07, p=0.288.

Percentile classification of the data indicates that in the 75th percentile, fractures in the supplemented group healed within 17 weeks, while those in placebo group healed in 19 weeks (Table 3).

**Table 3 - Effect of Supplementation on Bone Fracture HealingTime**

| **Criteria** | **Supplemented Group** | **Placebo Group** |
|---|---|---|
| Number of patients | 21 | 36 |
| Age range (years) | 15 to 65 | 12 to 75 |
| Mean age (years) | 35 | 32 |
| Healing time (weeks) | 14:0±1.1 | 16.9±1.2 |
| 75^{th} percentile healing period (weeks) | 17 | 19 |

The percentage of patients experiencing early fracture healing (in 10 weeks or less) differed by 33.3% in the supplemented group and 11.1% in the placebo group (Chi-square analysis=2.853, p=0.091). The percentage of patients that had delayed healing (more than 20 weeks) was 9.5% in the supplemented group and 19.4 % in the placebo group (Table 4).

**Table 4 - Distribution (Percentage) of Patients by Fracture Healing Time**

| **Healing Time** | **Supplemented Group** | **Placebo Group** |
|---|---|---|
| 10 weeks or less | 33.3% | 11.1% |
| 11 to 15 weeks | 33.3% | 52.8% |
| 16 to 20 weeks | 23.8% | 16.7% |
| More than 20 weeks | 9.5% | 19.4% |

The healing time of bone fracture in patients of different ages is shown in Table 6. In the patients of 10-20 years of age, the healing time reduced from 17.6 weeks to 9 weeks (reduced 49 %). In the patients of 31-40 years of age, the healing time reduced from 17.1 weeks to 10.7 weeks (reduced 37%). In the patients of 41-50 years of age, the healing time reduced from 21.2 weeks to 12.7 weeks (reduced 40%). In the patients of >50 years of age, the healing time reduced from 16 weeks to 15.7 weeks (reduced 1.8%). Overall, the healing time in the supplemented group, except in the 21-30 year old group, is reduced. (Table 5).

**Table 5 - Healing Time (in Weeks) for Various Age Groups**

| **Age Groups** | **10 -20 yr** | **21-30 yr** | **31-40 yr** | **41-50 yr** | **> 50 yr** |
|---|---|---|---|---|---|
| **Supplemented Group** | | | | | |
| **Healing Time** | | | | | |
| Mean (in wks) | 9 | 18.3 | 10.7 | 12.7 | 15.7 |
| Range (in wks) | 9 | 9-26 | 6-16 | 11-14 | 14-19 |
| | | | | | |
| **Placebo Group** | | | | | |
| **Healing Time** | | | | | |
| Mean (in wks) | 17.6 | 14.6 | 17.1 | 21.2 | 16 |
| Range (in wks) | 9-30 | 9-30 | 11-39 | 13-30 | 10-20 |
| | | | | | |

The data suggest that administering to patients suffering from bone fracture with the present nutritional composition at the specified doses effectively reduces the healing time by at least two weeks in 75% of the patients. Patients in the supplemented group also reported an enhanced feeling of general well being during the study. The strongest effects can be seen in the adolescent age group (i.e., 10 to 20 years of age) who has a 49% reduction in the healing time. Patients who are 41-50 years and 31-40 years of age also have a significant reduction in the healing time (i.e., 40 % and 37%, respectively). It is believed that patients in the other age groups may likely to receive the same benefits if the dosage of the nutritional supplementation is optimized.

There was no signficant difference in the blood calcium levels in the supplemented group as compared to that in the placebo group. All the patients were found to have their blood calcium levels within the normal range.

The reduction in healing time is believed to be due to the supplementation of key ingredients which comprise lysine, proline, ascorbic acid, copper and vitamin B₆, as well as additional ingredients used in the present study. The present data provide evidence that nutritional supplementation in patents suffering from bone fracture can facilitate the healing (i.e., reduce healing time). Administration of the present nutritional compositions to bone fractured patients would have a positive impact on early functional recovery, improved well being, reduced medical costs, and reduced cost to business.

### The figures show:

Figure 1 depicts a radiograph of tibial shaft fracture immediately prior to reduction.
Figure 2 depicts a radiograph of tibial shaft fracture at healing at 12 weeks.
Figure 3 depicts ascorbic acid levels (urinalysis) in supplemented (patient #1-29) and placebo (patient #30-70) groups.
Figure 4 depicts a distribution of patients (percentage) by tibial fracture healing time.

## Claims

1. A nutritional composition, comprising lysine, proline, ascorbic acid, copper, and vitamin B₆, wherein the nutritional composition comprises 27 - 34 % wt lysine, 14 - 16 % wt proline and 42 - 47 % wt ascorbic acid.

2. Nutritional composition according to claim 1, wherein the nutritional composition comprises 28 - 33 % wt lysine, 15 - 16 % wt proline and 43 - 46 % wt ascorbic acid.

3. Nutritional composition according to claim 1 or claim 2, wherein the nutritional composition provides a daily dosage of
a) 230 mg - 10 grams lysine, 120 mg - 5 grams proline, 360 mg - 15 grams ascorbic acid, 1.5 µg - 20 mg copper, and 0.2 mg - 20 mg vitamin B₆;
b) 1,010 mg - 8 grams lysine, 560 mg - 4 grams proline, 1,500 mg - 9 grams ascorbic acid, 2 µg - 6 mg copper, and 0.5 mg - 10 mg vitamin B₆; or
c) 1,010 mg lysine, 560 mg proline, 1,500 mg ascorbic acid, 330 µg copper and 10 mg vitamin B₆.

4. Nutritional composition according to one of the preceding claims, wherein said composition provides a daily dosage per body weight of
a) 3.2 - 139 mg/kg lysine, 1.7 - 69.4 mg/kg proline, 5 - 208.3 mg/kg ascorbic acid, 0.02 - 278 µg/kg copper, 2.78 - 279 µg/kg vitamin B₆;
b) 14 - 111 mg/kg lysine, 7.8 - 55.6 mg/kg proline, 20.8 - 125 mg/kg ascorbic acid, 0.03 - 83.3 µg/kg copper, and 6.94 - 139 µg/kg vitamin B₆; or
c) 14 mg/kg lysine, 7.8 mg/kg proline, 20.8 mg/kg ascorbic acid, 4.6 µg/kg copper, 139 µg/kg vitamin B₆.

5. Nutritional composition according to one of the preceding claims, wherein the nutritional composition further comprises vitamin A, vitamin D3, vitamin E, vitamin B1, vitamin B2, niacin, folic acid, vitamin B12, biotin, pantothenic acid, calcium, phosphorus, magnesium, zinc, selenium, manganese, chromium, molybdenum, potassium, citrus fruit peel bioflavanoids, arginine, cysteine, inositol, carnitine, coenzyme Q10, and pycnogenol.

6. Nutritional composition according to claim 5, wherein the nutritional composition provides a daily dosage of .
a) 67 µg - 100 mg vitamin A, 0.7 µg - 50 µg vitamin D₃, 0.7 µg - 50 µg vitamin E, 1.4 mg - 8 mg vitamin B₁, 1.4 mg - 8 mg vitamin B₂, 9 mg - 250 mg niacin, 18 µg - 500 µg folic acid, 4 µg - 100 µg vitamin B₁₂, 13 µg - 400 µg biotin, 8 mg - 100 mg pantothenic acid, 7 mg - 40 mg calcium, 3 mg - 300 mg phosphorus, 40 mg - 200 mg magnesium, 0.5 mg - 10 mg zinc, 20 µg - 300 µg selenium, 0.8 mg - 15 mg manganese, 2 µg - 200 µg chromium, 0.8 µg - 100 µg molybdenum, 4 mg - 300 mg potassium, 20 mg - 500 mg citrus fruit peel bioflavanoids, 10 mg - 500 mg arginine, 10 mg - 400 mg cysteine, 5 mg - 400 mg inositol, 5 mg - 400 mg carnitine, 1.6 mg - 70 mg coenzyme Q₁₀, and 1.6 mg - 70 mg pycnogenol;
b) 166 µg - 50 mg vitamin A, 1.65 µg - 20 µg vitamin D₃, 1.65 µg - 20 µg vitamin E, 3.5 mg - 7 mg vitamin B₁, 3.5 mg - 7 mg vitamin B₂, 22.5 mg - 100 mg niacin, 45 µg - 300 µg folic acid, 10 µg - 50 µg vitamin B₁₂, 32 µg - 300 µg biotin, 20 mg - 60 mg pantothenic acid, 17 mg - 35 mg calcium, 7 mg - 100 mg phosphorus, 50 mg - 100 mg magnesium, 3 mg - 8 mg zinc, 30 µg - 250 µg selenium, 1 mg - 3.25 mg manganese, 2 µg - 75 µg chromium, 2 µg - 75 µg molybdenum, 8 mg - 200 mg potassium, 50 mg - 250 mg citrus fruit peel bioflavanoids, 100 mg - 300 mg arginine, 80 mg - 200 mg cysteine, 80 mg - 200 mg inositol, 80 mg - 200 mg carnitine, 3 mg - 35 mg coenzyme Q₁₀, and 3 mg - 35 mg pycnogenol; or
c) 333 µg vitamin A, 3.3 µg vitamin D₃, 3.3 µg vitamin E, 7 mg vitamin B₁, 7 mg vitamin B₂, 45 mg niacin, 90 µg folic acid, 20 µg vitamin B₁₂, 65 µg biotin, 40 mg pantothenic acid, 35 mg calcium, 15 mg phosphorus, 40 mg magnesium, 7 mg zinc, 20 µg selenium, 1.3 mg manganese, 10 µg chromium, 4 µg molybdenum, 20 mg potassium, 100 mg citrus fruit peel bioflavanoids, 40 mg arginine, 35 mg cysteine, 35 mg inositol, 35 mg carnitine, 7 mg coenzyme Q₁₀, and 7 mg pycnogenol.

7. Nutritional composition according to claim 5 or claim 6, wherein said composition further comprises a daily dosage per body weight of
a) 0.9 - 1,390 µg/kg vitamin A, 0.01 - 0.694 µg/kg vitamin D₃, 0.01 - 0.694 µg/kg vitamin E, 19.4 - 111 µg/kg vitamin B₁, 19.4 - 111 µg/kg vitamin B₂, 125 - 3,472 µg/kg niacin, 0.25 - 6.94 µg/kg folic acid, 0.05 - 1.39 µg/kg vitamin B₁₂, 0.181 - 5.56 µg/kg biotin, 111 - 1,390 µg/kg pantothenic acid, 97.2 - 555 µg/kg calcium, 42 - 4,167 µg/kg phosphorus, 555 - 2,778 µg/kg magnesium, 6.9 -139 µg/kg zinc, 0.28 - 4.17 µg/kg selenium, 11.1 - 208.3 µg/kg manganese, 0.03 - 2.78. µg/kg chromium, 0.01. - 1.39 µg/kg molybdenum, 55.6 - 4,167 µg/kg potassium, 278 - 6,944 µg/kg citrus fruit peel bioflavanoids, 139 - 6,944 µg/kg arginine, 135-5,555 µg/kg cysteine, 69 - 5,555 µg/kg inositol, 69 - 5,555 µg/kg carnitine, 22.2 - 972 µg/kg coenzyme Q₁₀, and 22.2 - 972 µg/kg pycnogenol;
b) 2.31 - 694 µg/kg vitamin A, 0.023- 0.278 µg/kg vitamin D₃, 0.023 - 0.278 µg/kg vitamin E, 48.6 - 97.2 µg/kg vitamin B₁, 48.6 - 97.2 µg/kg vitamin B₂. 312.5 - 3,190 µg/kg niacin, 0.6 - 4.17 µg/kg folic acid, 0.14 - 0.69 µg/kg vitamin B₁₂, 0.444 - 4.17. µg/kg biotin, 278 - 833 µg/kg pantothenic acid, 236 - 903 µg/kg calcium, 97.2 - 1,390 µg/kg phosphorus, 694 - 1,390 µg/kg magnesium, 41.7 - 111 µg/kg zinc; 0.42 - 3.47 µg/kg selenium, 13.9 - 45.1 µg/kg manganese, 0.07 - 2.78 µg/kg chromium, 0.03 - 1.04 µg/kg molybdenum, 111.1 - 2,778 µg/kg potassium, 694 - 3,472 µg/kg citrus fruit peel bioflavanoids, 1,389 - 4,167 µg/kg arginine, 1,111 - 2,778 µg/kg cysteine, 1,111 - 2,778 µg/kg inositol, 1,111 - 2,778 µg/kg carnitine, 41.7 - 486 µg/kg coenzyme Q₁₀, and 41.7 - 486 µg/kg pycnogenol; or
c) 4.6 µg/kg vitamin A, 0.046 µg/kg vitamin D₃, 0.046 µg/kg vitamin E, 97.2 µg/kg vitamin B₁. 97.2 µg/kg vitamin B₂, 625 µg/kg niacin, 1.25 µg/kg folic acid, 0.27 µg/kg vitamin B₁₂, 0.9 µg/kg biotin, 555 µg/kg pantothenic acid, 486 µg/kg calcium, 208 µg/kg phosphorus, 555 µg/kg magnesium, 97.2 µg/kg zinc, 0.78 µg/kg selenium, 18.1 µg/kg manganese, 0.14 µg/kg chromium, 0.06 µg/kg molybdenum, 277.8 µg/kg potassium, 1,389 µg/kg citrus fruit peel bioflavanoids, 555 µg/kg arginine, 486 µg/kg cysteine, 486 µg/kg inositol, 486 µg/kg carnitine, 97.2 µg/kg coenzyme Q₁₀, and 97.2 µg/kg pycnogenol.

8. Use of the nutritional composition of any one of claims 1 to 7 for the preparation of a pharmaceutical composition.

9. Use of the nutritional composition of any one of claims 1 to 7 for the preparation of a pharmaceutical composition for facilitating bone healing in a mammal.

10. Use according to claim 9, wherein said mammal is a human.

11. Use according to claim 9 or claim 10, wherein said composition is to be administered orally, intravenously or parenterally.

## Patentansprüche

1. Nahrungsergänzungsmittelzusammensetzung, die Lysin, Prolin, Ascorbinsäure, Kupfer und Vitamin B₆ umfasst, wobei die Nahrungsergänzungsmittelzusammensetzung 27 - 34 Gew.-% Lysin, 14-16 Gew.-% Prolin und 42 - 47 Gew.-% Ascorbinsäure umfasst.

2. Nahrungsergänzungsmittelzusammensetzung nach Anspruch 1, wobei die Nahrungsergänzungsmittelzusammensetzung 28 - 33 Gew.-% Lysin, 15-16 Gew.-% Prolin und 43 - 46 Gew.-% Ascorbinsäure umfasst.

3. Nahrungsergänzungsmittelzusammensetzung nach Anspruch 1 oder Anspruch 2, wobei die Nahrungsergänzungsmittelzusammensetzung eine tägliche Dosis von
a) 230 mg - 10 g Lysin, 120 mg - 5 g Prolin, 360 mg - 15 g Ascorbinsäure, 1,5 µg - 20 mg Kupfer und 0,2 mg - 20 mg Vitamin B₆;
b) 1.010 mg - 8 g Lysin, 560 mg - 4 g Prolin, 1.500 mg - 9 g Ascorbinsäure, 2 µg - 6 mg Kupfer und 0,5 mg - 10 mg Vitamin B₆; oder
c) 1.010 mg Lysin, 560 mg Prolin, 1.500 mg Ascorbinsäure, 330 µg Kupfer und 10 mg Vitamin B₆
bereitstellt.

4. Nahrungsergänzungsmittelzusammensetzung nach einem der vorhergehenden Ansprüche, wobei die Zusammensetzung eine tägliche Dosis pro Körpergewicht von
a) 3,2 - 139 mg/kg Lysin, 1,7 - 69,4 mg/kg Prolin, 5 - 208,3 mg/kg Ascorbinsäure, 0,02 - 278 µg/kg Kupfer, 2,78 - 279 µg/kg Vitamin B₆;
b) 14 - 111 mg/kg Lysin, 7,8 - 55,6 mg/kg Prolin, 20,8 - 125 mg/kg Ascorbinsäure, 0,03 - 83,3 µg/kg Kupfer und 6,94 - 139 µg/kg Vitamin B₆; oder
c) 14 mg/kg Lysin, 7,8 mg/kg Prolin, 20,8 mg/kg Ascorbinsäure, 4,6 µg/kg Kupfer, 139 µg/kg Vitamin B₆
bereitstellt.

5. Nahrungsergänzungsmittelzusammensetzung nach einem der vorhergehenden Ansprüche, wobei die Nahrungsergänzungsmittelzusammensetzung weiterhin Vitamin A, Vitamin D₃, Vitamin E, Vitamin B₁, Vitamin B₂, Niacin, Folsäure, Vitamin B₁₂, Biotin, Pantothensäure, Kalzium, Phosphor, Magnesium, Zink, Selen, Mangan, Chrom, Molybdän, Kalium, Bioflavonoide von Zitrusfruchtschalen, Arginin, Cystein, Inositol, Carnitin, Coenzym Q₁₀ und Pycnogenol umfasst.

6. Nahrungsergänzungsmittelzusammensetzung nach Anspruch 5, wobei die Nahrungsergänzungsmittelzusammensetzung eine tägliche Dosis von
a) 67 µg - 100 mg Vitamin A, 0,7 µg - 50 µg Vitamin D₃, 0,7 µg - 50 µg Vitamin E, 1,4 mg - 8 mg Vitamin B₁, 1,4 mg - 8 mg Vitamin B₂, 9 mg - 250 mg Niacin, 18 µg - 500 µg Folsäure, 4 µg - 100 µg Vitamin B₁₂, 13 µg - 400 µg Biotin, 8 mg - 100 mg Pantothensäure, 7 mg - 40 mg Kalzium, 3 mg - 300 mg Phosphor, 40 mg - 200 mg Magnesium, 0,5 mg - 10 mg Zink, 20 µg - 300 µg Selen, 0,8 mg - 15 mg Mangan, 2 µg - 200 µg Chrom, 0,8 µg - 100 µg Molybdän, 4 mg - 300 mg Kalium, 20 mg - 500 mg Bioflavonoide von Zitrusfruchtschalen, 10 mg - 500 mg Arginin, 10 mg - 400 mg Cystein, 5 mg - 400 mg Inositol, 5 mg - 400 mg Carnitin, 1,6 mg - 70 mg Coenzym Q₁₀ und 1,6 mg - 70 mg Pycnogenol;
b) 166 µg - 50 mg Vitamin A, 1,65 µg - 20 µg Vitamin D₃, 1,65 µg - 20 µg Vitamin E, 3,5 mg - 7 mg Vitamin B₁, 3,5 mg - 7 mg Vitamin B₂, 22,5 mg - 100 mg Niacin, 45 µg - 300 µg Folsäure, 10 µg - 50 µg Vitamin B₁₂, 32 µg - 300 µg Biotin, 20 mg - 60 mg Pantothensäure, 17 mg - 35 mg Kalzium, 7 mg - 100 mg Phosphor, 50 mg - 100 mg Magnesium, 3 mg - 8 mg Zink, 30 µg - 250 µg Selen, 1 mg - 3,25 mg Mangan, 2 µg - 75 µg Chrom, 2 µg - 75 µg Molybdän, 8 mg - 200 mg Kalium, 50 mg - 250 mg Bioflavonoide von Zitrusfruchtschalen, 100 mg - 300 mg Arginin, 80 mg - 200 mg Cystein, 80 mg - 200 mg Inositol, 80 mg - 200 mg Carnitin, 3 mg - 35 mg Coenzym Q₁₀ und 3 mg - 35 mg Pycnogenol; oder
c) 333 µg Vitamin A, 3,3 µg Vitamin D₃, 3,3 µg Vitamin E, 7 mg Vitamin B₁, 7 mg Vitamin B₂, 45 mg Niacin, 90 µg Folsäure, 20 µg Vitamin B₁₂, 65 µg Biotin, 40 mg Pantothensäure, 35 mg Kalzium, 15 mg Phosphor, 40 mg Magnesium, 7 mg Zink, 20 µg Selen, 1,3 mg Mangan, 10 µg Chrom, 4 µg Molybdän, 20 mg Kalium, 100 mg Bioflavonoide von Zitrusfruchtschalen, 40 mg Arginin, 35 mg Cystein, 35 mg Inositol, 35 mg Carnitin, 7 mg Coenzym Q₁₀ und 7 mg Pycnogenol
bereitgestellt.

7. Nahrungsergänzungsmittelzusammensetzung nach Anspruch 5 oder Anspruch 6, wobei die Nahrungsergänzungsmittelzusammensetzung weiterhin eine tägliche Dosis pro Körpergewicht von
a) 0,9 - 1.390 µg/kg Vitamin A, 0,01 - 0,694 µg/kg Vitamin D₃, 0,01 - 0,694 µg/kg Vitamin E, 19,4 - 111 µg/kg Vitamin B₁, 19,4 - 111 µg/kg Vitamin B₂, 125 - 3.472 µg/kg Niacin, 0,25 - 6,94 µg/kg Folsäure, 0,05 - 1,39 µg/kg Vitamin B₁₂, 0,181 - 5,56 µg/kg Biotin, 111 - 1.390 µg/kg Pantothensäure, 97,2 - 555 µg/kg Kalzium, 42 - 4.167 µg/kg Phosphor, 555 - 2.778 µg/kg Magnesium, 6,9 - 139 µg/kg Zink, 0,28 - 4,17 µg/kg Selen, 11,1 - 208,3 µg/kg Mangan, 0,03 - 2,78 µg/kg Chrom, 0,01 - 1,39 µg/kg Molybdän, 55,6 - 4.167 µg/kg Kalium, 278 - 6.944 µg/kg Bioflavonoide von Zitrusfruchtschalen, 139 - 6.944 µg/kg Arginin, 135 - 5.555 µg/kg Cystein, 69 - 5.555 µg/kg Inositol, 69 - 5.555 µg/kg Carnitin, 22,2 - 972 µg/kg Coenzym Q₁₀ und 22,2 - 972 µg/kg Pycnogenol;
b) 2,31 - 694 µg/kg Vitamin A, 0,023 - 0,278 µg/kg Vitamin D₃, 0,023 - 0,278 µg/kg Vitamin E, 48,6 - 97,2 µg/kg Vitamin B₁, 48,6 - 97,2 µg/kg Vitamin B₂, 312,5 - 3.190 µg/kg Niacin, 0,6 - 4,17 µg/kg Folsäure, 0,14 - 0,69 µg/kg Vitamin B₁₂, 0,444 - 4,17 µg/kg Biotin, 278 - 833 µg/kg Pantothensäure, 236 - 903 µg/kg Kalzium, 97,2 - 1.390 µg/kg Phosphor, 694 - 1.390 µg/kg Magnesium, 41,7 - 111 µg/kg Zink, 0,42 - 3,47 µg/kg Selen, 13,9 - 45,1 µg/kg Mangan, 0,07 - 2,78 µg/kg Chrom, 0,03 - 1,04 µg/kg Molybdän, 111,1 - 2.778 µg/kg Kalium, 694 - 3.472 µg/kg Bioflavonoide von Zitrusfruchtschalen, 1.389 - 4.167 µg/kg Arginin, 1.111 - 2.778 µg/kg Cystein, 1.111 - 2.778 µg/kg Inositol, 1.111 - 2.778 µg/kg Carnitin, 41,7 - 486 µg/kg Coenzym Q₁₀ und 41,7 - 486 µg/kg Pycnogenol; oder
c) 4,6 µg/kg Vitamin A, 0,046 µg/kg Vitamin D₃, 0,046 µg/kg Vitamin E, 97,2 µg/kg Vitamin B₁, 97,2 µg/kg Vitamin B₂, 625 µg/kg Niacin, 1,25 µg/kg Folsäure, 0,27 µg/kg Vitamin B₁₂, 0,9 µg/kg Biotin, 555 µg/kg Pantothensäure, 486 µg/kg Kalzium, 208 µg/kg Phosphor, 555 µg/kg Magnesium, 97,2 µg/kg Zink, 0,78 µg/kg Selen, 18,1 µg/kg Mangan, 0,14 µg/kg Chrom, 0,06 µg/kg Molybdän, 277,8 µg/kg Kalium, 1.389 µg/kg Bioflavonoide von Zitrusfruchtschalen, 555 µg/kg Arginin, 486 µg/kg Cystein, 486 µg/kg Inositol, 486 µg/kg Carnitin, 97,2 µg/kg Coenzym Q₁₀ und 97,2 µg/kg Pycnogenol
umfasst.

8. Verwendung der Nahrungsergänzungsmittelzusammensetzung gemäß einem der Ansprüche 1 bis 7 für die Herstellung einer pharmazeutischen Zusammensetzung.

9. Verwendung der Nahrungsergänzungsmittelzusammensetzung gemäß einem der Ansprüche 1 bis 7 für die Herstellung einer pharmazeutischen Zusammensetzung, die die Knochenheilung in Säugetieren erleichtert.

10. Verwendung nach Anspruch 9, wobei das Säugetier ein Mensch ist.

11. Verwendung nach Anspruch 9 oder Anspruch 10, wobei die Nahrungsergänzungsmittelzusammensetzung oral, intravenös oder parenteral zu verabreichen ist.

## Revendications

1. Composition nutritionnelle comprenant de la lysine, de la proline, de l'acide ascorbique, du cuivre et de la vitamine B₆, la composition nutritionnelle comprenant de 27 à 34 % en poids de lysine, de 14 à 16 % en poids de proline et de 42 à 47 % en poids d'acide ascorbique.

2. Composition nutritionnelle selon la revendication 1, la composition nutritionnelle comprenant de 28 à 33 % en poids de lysine, de 15 à 16 % en poids de proline et de 43 à 46 % en poids d'acide ascorbique.

3. Composition nutritionnelle selon la revendication 1 ou la revendication 2, la composition nutritionnelle apportant une dose journalière de :
a) 230 mg à 10 g de lysine, 120 mg à 5 g de proline, 360 mg à 15 g d'acide ascorbique, 1,5 µg à 20 mg de cuivre et 0,2 mg à 20 mg de vitamine B₆ ;
b) 1 010 mg à 8 g de lysine, 560 mg à 4 g de proline, 1 500 mg à 9 g d'acide ascorbique, 2 µg à 6 mg de cuivre et 0,5 mg à 10 mg de vitamine B₆ ; ou
c) 1 010 mg de lysine, 560 mg de proline, 1 500 mg d'acide ascorbique, 330 µg de cuivre et 10 mg de vitamine B₆ .

4. Composition nutritionnelle selon l'une des revendications précédentes, ladite composition apportant une dose journalière par poids corporel de :
a) 3,2 à 139 mg/kg de lysine, 1,7 à 69,4 mg/kg de proline, 5 à 208,3 mg/kg d'acide ascorbique, 0,02 à 278 µg/kg de cuivre et 2,78 à 279 µg/kg de vitamine B₆ ;
b) 14 à 111 mg/kg de lysine, 7,8 à 55,6 mg/kg de proline, 20,8 à 125 mg/kg d'acide ascorbique, 0,03 à 83,3 µg/kg de cuivre et 6,94 à 139 µg/kg de vitamine B₆ ; ou
c) 14 mg/kg de lysine, 7,8 mg/kg de proline, 20,8 mg/kg d'acide ascorbique, 4,6 µg/kg de cuivre et 139 µg/kg de vitamine B₆ .

5. Composition nutritionnelle selon l'une des revendications précédentes, la composition nutritionnelle comprenant en outre de la vitamine A, de la vitamine D₃, de la vitamine E, de la vitamine B₁, de la vitamine B₂, de la niacine, de l'acide folique, de la vitamine B₁₂, de la biotine, de l'acide pantothénique, du calcium, du phosphore, du magnésium, du zinc, du sélénium, du manganèse, du chrome, du molybdène, du potassium, des bioflavonoïdes d'écorce d'agrumes, de l'arginine, de la cystéine, de l'inositol, de la carnitine, de la coenzyme Q₁₀ et du pycnogénol.

6. Composition nutritionnelle selon la revendication 5, la composition nutritionnelle apportant une dose journalière de :
a) 67 µg à 100 mg de vitamine A, 0,7 µg à 50 µg de vitamine D₃, 0,7 µg à 50 µg de vitamine E, 1,4 mg à 8 mg de vitamine B₁, 1,4 mg à 8 mg de vitamine B₂, 9 mg à 250 mg de niacine, 18 µg à 500 µg d'acide folique, 4 µg à 100 µg de vitamine B₁₂, 13 µg à 400 µg de biotine, 8 mg à 100 mg d'acide pantothénique, 7 mg à 40 mg de calcium, 3 mg à 300 mg de phosphore, 40 mg à 200 mg de magnésium, 0,5 mg à 10 mg de zinc, 20 µg à 300 µg de sélénium, 0,8 mg à 15 mg de manganèse, 2 µg à 200 µg de chrome, 0,8 µg à 100 µg de molybdène, 4 mg à 300 mg de potassium, 20 mg à 500 mg de bioflavonoïdes d'écorce d'agrumes, 10 mg à 500 mg d'arginine, 10 mg à 400 mg de cystéine, 5 mg à 400 mg d'inositol, 5 mg à 400 mg de carnitine, 1,6 mg à 70 mg de coenzyme Q₁₀ et 1,6 mg à 70 mg de pycnogénol ;
b) 166 µg à 50 mg de vitamine A, 1,65 µg à 20 µg de vitamine D₃, 1,65 µg à 20 µg de vitamine E, 3,5 mg à 7 mg de vitamine B₁, 3,5 mg à 7 mg de vitamine B₂, 22,5 mg à 100 mg de niacine, 45 µg à 300 µg d'acide folique, 10 µg à 50 µg de vitamine B₁₂, 32 µg à 300 µg de biotine, 20 mg à 60 mg d'acide pantothénique, 17 mg à 35 mg de calcium, 7 mg à 100 mg de phosphore, 50 mg à 100 mg de magnésium, 3 mg à 8 mg de zinc, 30 µg à 250 µg de sélénium, 1 mg à 3,25 mg de manganèse, 2 µg à 75 µg de chrome, 2 µg à 75 µg de molybdène, 8 mg à 200 mg de potassium, 50 mg à 250 mg de bioflavonoïdes d'écorce d'agrumes, 100 mg à 300 mg d'arginine, 80 mg à 200 mg de cystéine, 80 mg à 200 mg d'inositol, 80 mg à 200 mg de carnitine, 3 mg à 35 mg de coenzyme Q₁₀ et 3 mg à 35 mg de pycnogénol ; ou
c) 333 µg de vitamine A, 3,3 µg de vitamine D₃, 3,3 µg de vitamine E, 7 mg de vitamine B₁, 7 mg de vitamine B₂, 45 mg de niacine, 90 µg d'acide folique, 20 µg de vitamine B₁₂, 65 µg de biotine, 40 mg d'acide pantothénique, 35 mg de calcium, 15 mg de phosphore, 40 mg de magnésium, 7 mg de zinc, 20 µg de sélénium, 1,3 mg de manganèse, 10 µg de chrome, 4 µg de molybdène, 20 mg de potassium, 100 mg de bioflavonoïdes d'écorce d'agrumes, 40 mg d'arginine, 35 mg de cystéine, 35 mg d'inositol, 35 mg de carnitine, 7 mg de coenzyme Q₁₀ et 7 mg de pycnogénol.

7. Composition nutritionnelle selon la revendication 5 ou la revendication 6, ladite composition comprenant en outre une dose journalière par poids corporel de :
a) 0,9 à 1 390 µg/kg de vitamine A, 0,01 à 0,694 µg/kg de vitamine D₃, 0,01 à 0,694 µg/kg de vitamine E, 19,4 à 111 µg/kg de vitamine B₁, 19,4 à 111 µg/kg de vitamine B₂, 125 à 3 472 µg/kg de niacine, 0,25 à 6,94 µg/kg d'acide folique, 0,05 à 1,39 µg/kg de vitamine B₁₂, 0,181 à 5,56 µg/kg de biotine, 111 à 1 390 µg/kg d'acide pantothénique, 97,2 à 555 µg/kg de calcium, 42 à 4 167 µg/kg de phosphore, 555 à 2 778 µg/kg de magnésium, 6,9 à 139 µg/kg de zinc, 0,28 à 4,17 µg/kg de sélénium, 11,1 à 208,3 µg/kg de manganèse, 0,03 à 2,78 µg/kg de chrome, 0,01 à 1,39 µg/kg de molybdène, 55,6 à 4 167 µg/kg de potassium, 278 à 6 944 µg/kg de bioflavonoïdes d'écorce d'agrumes, 139 à 6 944 µg/kg d'arginine, 135 à 5 555 µg/kg de cystéine, 69 à 5 555 µg/kg d'inositol, 69 à 5 555 µg/kg de carnitine, 22,2 à 972 µg/kg de coenzyme Q₁₀ et 22,2 à 972 µg/kg de pycnogénol ;
b) 2,31 à 694 µg/kg de vitamine A, 0,023 à 0,278 µg/kg de vitamine D₃, 0,023 à 0,278 µg/kg de vitamine E, 48,6 à 97,2 µg/kg de vitamine B₁, 48,6 à 97,2 µg/kg de vitamine B₂, 312,5 à 3 190 µg/kg de niacine, 0,6 à 4,17 µg/kg d'acide folique, 0,14 à 0,69 µg/kg de vitamine B₁₂, 0,444 à 4,17 µg/kg de biotine, 278 à 833 µg/kg d'acide pantothénique, 236 à 903 µg/kg de calcium, 97,2 à 1 390 µg/kg de phosphore, 694 à 1 390 µg/kg de magnésium, 41,7 à 111 µg/kg de zinc, 0,42 à 3,47 µg/kg de sélénium, 13,9 à 45,1 µg/kg de manganèse, 0,07 à 2,78 µg/kg de chrome, 0,03 à 1,04 µg/kg de molybdène, 111,1 à 2 778 µg/kg de potassium, 694 à 3 472 µg/kg de bioflavonoïdes d'écorce d'agrumes, 1 389 à 4 167 µg/kg d'arginine, 1 111 à 2 778 µg/kg de cystéine, 1 111 à 2 778 µg/kg d'inositol, 1 111 à 2 778 µg/kg de carnitine, 41,7 à 486 µg/kg de coenzyme Q₁₀ et 41,7 à 486 µg/kg de pycnogénol ; ou
c) 4,6 µg/kg de vitamine A, 0,046 µg/kg de vitamine D₃, 0,046 µg/kg de vitamine E, 97,2 µg/kg de vitamine B₁, 97,2 µg/kg de vitamine B₂, 625 µg/kg de niacine, 1,25 µg/kg d'acide folique, 0,27 µg/kg de vitamine B₁₂, 0,9 µg/kg de biotine, 555 µg/kg d'acide pantothénique, 486 µg/kg de calcium, 208 µg/kg de phosphore, 555 µg/kg de magnésium, 97,2 µg/kg de zinc, 0,78 µg/kg de sélénium, 18,1 µg/kg de manganèse, 0,14 µg/kg de chrome, 0,06 µg/kg de molybdène, 277,8 µg/kg de potassium, 1 389 µg/kg de bioflavonoïdes d'écorce d'agrumes, 555 µg/kg d'arginine, 486 µg/kg de cystéine, 486 µg/kg d'inositol, 486 µg/kg de carnitine, 97,2 µg/kg de coenzyme Q₁₀ et 97,2 µg/kg de pycnogénol.

8. Utilisation de la composition nutritionnelle selon l'une quelconque des revendications 1 à 7, pour la préparation d'une composition pharmaceutique.

9. Utilisation de la composition nutritionnelle selon l'une quelconque des revendications 1 à 7, pour la préparation d'une composition pharmaceutique permettant de faciliter la cicatrisation osseuse chez un mammifère.

10. Utilisation selon la revendication 9, ledit mammifère étant un être humain.

11. Utilisation selon la revendication 9 ou la revendication 10, ladite composition devant être administrée par voie orale, intraveineuse ou parentérale.
